# EUROPEAN PATENT SPECIFICATION

(11) **EP 4 051 358 B1**
(45) Date of publication and mention of the grant of the patent: **10.09.2025**
(21) Application number: 20811181.5
(22) Date of filing: 27.10.2020
(51) Int. Cl.: A61L 29/12, A61M 25/01, A61M 25/00, A61L 29/14, A61L 29/16

(54) **URINARY CATHETERS AND METHODS FOR PREVENTING BACTERIAL INFECTIONS**
HARNKATHETER UND VERFAHREN ZUM VERHINDERN BAKTERIELLER INFEKTIONEN
CATHÉTERS URINAIRES ET PROCÉDÉS POUR PRÉVENIR DES INFECTIONS BACTÉRIENNES

(30) Priority: 28.10.2019 US 201962926912 P
(43) Date of publication of application: 07.09.2022
(73) Proprietor: Hollister Incorporated, Libertyville IL 60048 (US)
(72) Inventor: SILEIKA, Tadas S., Libertyville, IL 60048 (US); DEAN, Nicole L., Libertyville, IL 60048 (US); PETTIGREW, Jacqueline MJ, Libertyville, IL 60048 (US)
(74) Representative: FRKelly
(86) International application number: PCT/US2020/057530
(87) International publication number: WO 2021/086851

(56) References cited:
- WO-A1-2009/096822
- WO-A1-2012/116209
- KR-A- 20180 034 848
- US-A1- 2019 015 640

## Description

The present application claims the benefit of and priority to U.S. Provisional Application No. 62/926,912, filed October 28,2019.

### TECHNICAL FIELD

The present disclosure generally relates to urinary catheters and methods for preventing bacterial infections, and more particularly, to catheters and methods for preventing urinary tract infections. The present disclosure also relates to urinary catheters and methods that hinder the motility structures of bacteria within the urethra.

### BACKGROUND

It is desirable for urinary catheters to have a lubricated or lubricious outer surface to increase comfort of the patient during insertion into and/or removal from the body. One method for rendering the surface of a urinary catheter lubricious is to coat at least the insertable portion of the catheter with a lubricious hydrophilic coating. Another method is to apply a lubricant to the urinary catheter. Lubricity of the catheter minimizes soft tissue damage and reduces overall discomfort during use of the medical device.

Although catheters are typically prepared in sterile environments and are provided with safe handling instructions, catheter users are at risk of contracting a urinary tract infection due to several different factors. For example, the catheter may become contaminated during use and introduce bacteria into the urethra. The catheter also may carrier bacteria along the urinary tract. Additionally, because urine is voided from the bladder through the catheter, urine does not flow directly through the urethra. Thus, urine is no longer a factor in flushing out the urethra or wetting of the urethral tissue.

Therefore, there is a need for catheters which reduce the risk of urinary tract infections.

### SUMMARY

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter product for insertion into the urethra. The urinary catheter product includes a catheter and a chemotactant. The catheter product is configured to deliver the chemotactant into the urethra.
Fig. 1 is a schematic illustration of a male urinary tract, shown with a gradient of chemotactant within the urethra;
Fig. 2 is a schematic illustration of a male urinary tract, shown with a bolus of chemotactant near the opening of the urethra;
Fig. 3 is a side elevational view, partially in section, of a catheter product of the present disclosure; and
Fig. 4 is a side elevational view of a catheter of the present disclosure.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

For catheter users, whether in-dwelling or intermittent, urine is no longer a factor that participates in regular flushing or wetting of the urethra. Because the users are unable to void directly through the urethra, the urethra no longer experiences the continuous flushing and re-wetting of the tissues that normally occurs during active voluntary urination. When the mechanism fails, the risk of urinary tract infections increases.

Urinary tract infections can be a result of bacteria swarming and/or traveling up through the urethra towards the bladder. Traveling of bacteria up the urethra is typically not a concern for those who can perform voluntary urination, because normal voiding tends to wash out and eliminate the bacteria from urethra, thereby prohibiting it from traveling toward the bladder and causing infection.

There are certain species of bacteria that are considered good "swimmers," which swarm and move when they receive certain signals from the surrounding environment. These bacteria are capable of movement even on solid surfaces through excretion of their own extracellular fluids. These motile bacteria also are known to become more pathogenic and increase their motility organelles, such as flagella (a process called hyperflagellation), when sensing certain signals, such as decreased viscosity in the fluids and mucus in their surrounding environment and/or an increase in chemotactants.

In a non-voiding patient, the urethral environment will be fairly viscous, due to the presence of mucosal secretions. When a hydrophilic catheter or a catheter lubricated with a water-based gel is introduced into the urethra, such introduction will likely deposit additional moisture, diluting the secretions, thus decreasing the overall viscosity of the urethral mucus and the surrounding urethral environment. This decrease in the urethral mucus viscosity provides a signal that activates the motility of the bacteria.

Furthermore, when a catheter is inserted into the urethra, the catheter, hydrophilic coating, hydration medium and/or lubricants may include a chemotactant that promotes chemotaxis (the movement of the bacteria in response to a chemical stimulant). For example, the hydrophilic coating and hydration medium may include a chemotactant, such as a polyol or polysaccharide, which stimulates the bacteria to move toward the chemotactant. If the catheter distributes this chemotactant at or near the bladder, the bacteria may be stimulated to undesirably swarm and move toward the bladder.

The catheter products disclosed herein are configured to deliver chemotactants within the urethra in a manner that guides the bacteria away from the bladder and back towards the distal urethral opening. For example, the catheter products may instill or form a chemotactic gradient within the urethra, wherein the chemotactic gradient promotes chemotaxis in a direction away from the bladder. The chemotactants may be substances or compounds to which bacteria is attracted. For example, the chemotactants may be a food or carbon source.

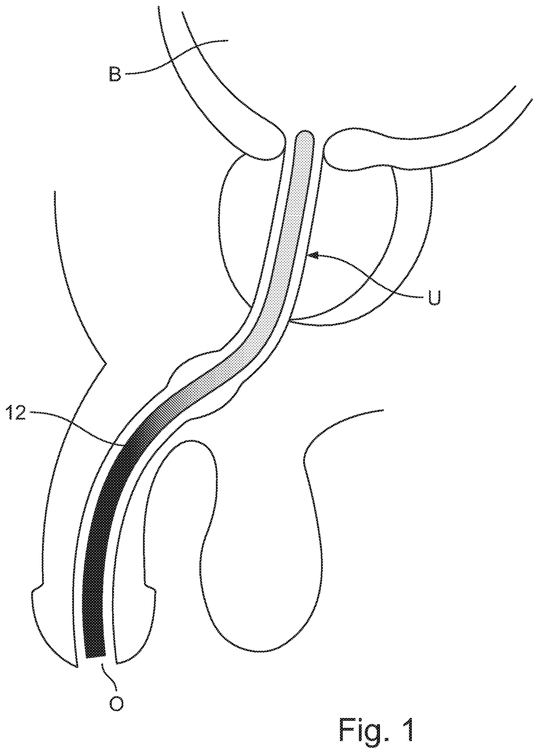

Fig. 1 schematically illustrates a male urethra U in which a catheter product has instilled a chemotactic gradient 12. In this figure, the chemotactic gradient 12 is represented by a line. The line has a relatively opaque appearance in the location where a larger amount of chemotactant is present and a relatively less opaque appearance in the location where a lesser amount of chemotactant is present. As illustrated in this figure, a larger amount or bolus of chemotactant is present near the opening O of the urethra U, and a lesser amount of chemotactant is present near the bladder B. Additionally, the amount of chemotactant gradually lessens from the opening O to the bladder B to create a gradient.

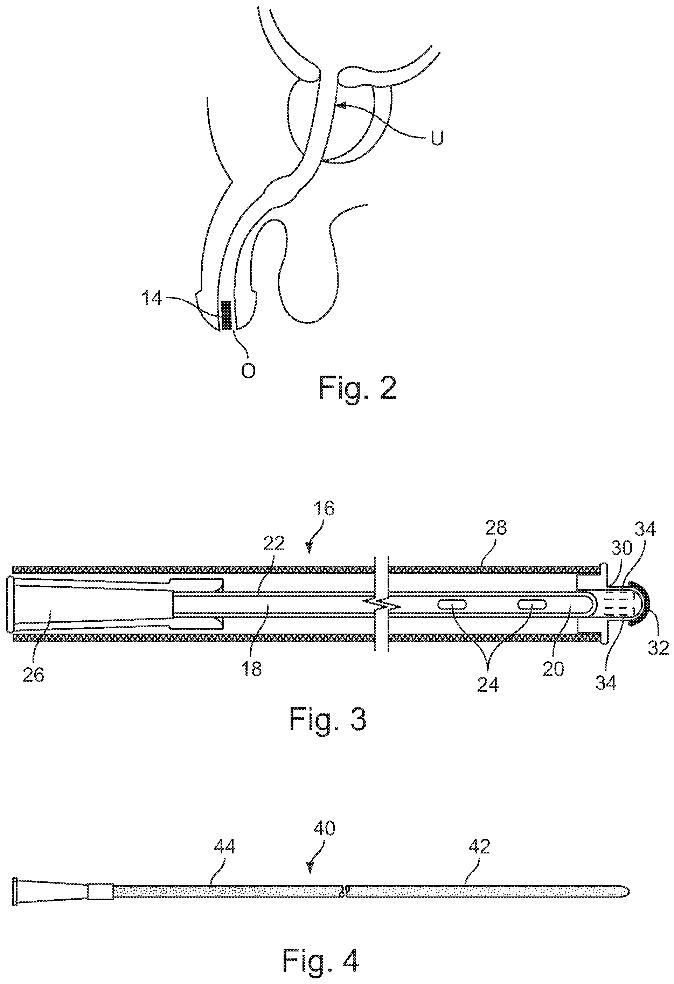

Turning to Fig. 2, this figure illustrates a male urethra U wherein the chemotactic gradient 14 includes an amount or bolus of chemotactant that has been deposited near the opening O of the urethra U and wherein the chemotactant does not extend far past the opening O.

In both Fig. 1 and Fig. 2, each of the gradients 12 and 14 creates a chemotactic signal sensed by bacteria that triggers motility and swarming behavior towards the larger amount or bolus of chemotactant.

The chemotactant may be any substance to which the bacteria is attracted and/or activates motility and swarming of the bacteria toward the substance. For example, the chemotactant may be sugar molecule (such as glucose, trehalose, sucrose, etc.), a polysaccharide (xanthan gum), or a polyol (glycerol), or another carbon source or chemotactant.

Turning now to Fig. 3, in one embodiment of a catheter product 16, the product includes a catheter 18 having proximal end 20 and a distal end 22. The proximal end 20 includes drainage openings 24 and the distal end 22 has a drainage member 26 associated therewith. Optionally, the catheter product 16 may include a sheath 28 for handling the catheter 18. The catheter product 16 may also include an introducer tip 30. During catheterization, the introducer tip 30 is inserted into the opening O of the urethra U and then the catheter 18 is inserted into the urethra U through the introducer tip 30.

The introducer tip 30 may be configured to deliver a chemotactant into the urethra U. In the illustrated embodiment, the introducer tip 30 includes a chemotactant 32 on the outer surface thereof. In one embodiment introducer tip 30 includes a bolus of chemotactant 32, such as a bolus of polyol or polysaccharide. The polyol may be for example glycerol. When the introducer tip 30 is inserted into the opening of the urethra, the introducer tip 30 deposits a bolus of chemotactant in the urethra at or near the urethral opening, similar to that shown in Fig. 2. Furthermore, the introducer tip 30 and catheter 18 may be configured such that as the catheter 18 is advanced through the introducer tip 30, the catheter 18 picks up some of the chemotactant and deposits along the urethra to create a chemotactic gradient, similar to that shown in Fig. 1.

In an alternative embodiment, in addition to coating the outer surface of the introducer tip 30 with a chemotactant, or in an alternative to this, the introducer tip 30 could include one or more reservoirs 34 that contain the chemotactant. In one embodiment, the chemotactant would be deployed from the reservoir 34 and into the urethral opening. In another embodiment, the chemotactant would be deployed from the reservoir 34 and onto the catheter 18 wherein the catheter would deposit the chemotactant along the urethra.

When a hydrophilic catheter is extended through the introducer tip 30, through the urethra, into the bladder and ultimately pulled out, residual moisture is left behind from the hydrophilic hydration medium, interacting not only with the middle and proximal portions of the urethra, but also interfacing with the bolus of the concentrated chemotactant left at the distal segment. Effectively, the chemotactant bolus then begins to slowly dissolve into the residual urethral moisture (either natural or from the residual hydration solution that is left behind). As the chemotactant dissolves, it begins to diffuse throughout the urethra, creating a concentration gradient: most concentrated at the urethral opening and least concentrated at the proximal portion of the urethra near the bladder. The gradient creates a chemotactic signal for bacteria, triggering motility and swarming behavior towards the chemotactant bolus. As the bacteria swim towards the bolus, they are brought away from the bladder, reducing the risk of infection in the bladder (and thus, UTIs). Additionally, it is know that some chemotactants are lethal to bacteria in high doses. Glycerol is one such chemotactant. Thus, it is possible that a bolus of glycerol would kill bacteria once bacteria get critically close to the bolus.

Referring not to Fig. 4, this figure illustrates a catheter 40 having sequential loading of a chemotactant. For example, when the catheter 40 includes a hydrophilic coating, the chemotactant may be loaded into the coating in a manner that results in a chemotactic gradient within the urethra during catheterization. The proximal end 42 of the catheter 40 (which would ultimately be inserted into the bladder) would include a lesser amount of a chemotactic agent, while the more distal portion 44 (closest to the funnel) should have the greatest amount of a chemotactic agent. Therefore, along the length of the catheter 40, the coating is embedded with a chemotactant at a gradient, increasing in concentration from the tip to the funnel. Upon insertion into the urethra, the chemotactant is deposited into the urethra by leaching out in the urethral environment.

Furthermore, the distal portion 44 of the catheter 40 closest to the funnel can also be instilled with an antimicrobial compound that is left behind in a distal segment of the urethra. The compound could be an antimicrobial polysaccharide (such as chitosan), or antimicrobial polyphenolic molecules (such as tannic acid, gallic acid, pyrogallol, etc.). Therefore, the antimicrobial gradient created by a non-antimicrobial chemotactants would result in swarming of bacteria towards an antimicrobial agent deposited at the distal urethra.

It will be understood that the embodiments described above are illustrative of some of the applications of the principles of the present subject matter. Numerous modifications may be made by those skilled in the art without departing from the scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A urinary catheter product (16) for insertion into the urethra, the urinary catheter product (16) comprising:
a catheter (18, 40) including a catheter shaft having a proximal end portion (20, 42) and a distal end portion (22, 44);
a chemotactant disposed on the catheter shaft; and
**characterized in that**;
the
amount of chemotactant disposed at the distal end portion (22, 44) of the catheter shaft is larger than an amount of chemotactant disposed at the proximal end portion (20, 42) of the catheter, so that the catheter product is configured to create a chemotactic gradient within the urethra.

2. The urinary catheter product of claim 1, wherein the catheter product further includes an introducer tip (30) and the chemotactant also is associated with the introducer tip.

3. The urinary catheter product of claim 2, wherein chemotactant is coated on a surface of the introducer tip (30).

4. The urinary catheter product of any one of claims 2 and 3, wherein the introducer tip (30) includes one or more reservoirs (34) and chemotactant is contained within the one or more reservoirs (34).

5. The urinary catheter product of claim 4, wherein the introducer tip (30) is configured to deploy chemotactant from the reservoir (34) onto the catheter (18) as the catheter (18) passes through the introducer tip (30).

6. The urinary catheter product of claim 5, wherein the introducer tip (30) is configured to deploy chemotactant from the reservoir (34) into the urethra.

7. The urinary catheter product of any one of claims 1-6, further including an antimicrobial associated with the distal end portion (22) of the catheter (18).

8. The urinary catheter product of any one of claims 1-7, wherein the catheter shaft includes a hydrophilic coating thereon, and the chemotactant is loaded into the hydrophilic coating.

## Patentansprüche

1. Harnkatheterprodukt (16) zur Einführung in die Harnröhre, wobei das Harnkatheterprodukt (16) Folgendes umfasst:
einen Katheter (18, 40), der einen Katheterschaft beinhaltet, der einen proximalen Endabschnitt (20, 42) und einen distalen Endabschnitt (22, 44) aufweist;
ein Chemotaktikum, das auf dem Katheterschaft angeordnet ist; und
**dadurch gekennzeichnet, dass**:
die Menge an Chemotaktikum, die an dem distalen Endabschnitt (22, 44) des Katheterschafts angeordnet ist, größer ist als eine Menge an Chemotaktikum, die an dem proximalen Endabschnitt (20, 42) des Katheters angeordnet ist, sodass das Katheterprodukt zum Erstellen eines chemotaktischen Gradienten innerhalb der Harnröhre konfiguriert ist.

2. Harnkatheterprodukt nach Anspruch 1, wobei das Katheterprodukt ferner eine Einführspitze (30) beinhaltet und das Chemotaktikum auch der Einführspitze zugeordnet ist.

3. Harnkatheterprodukt nach Anspruch 2, wobei das Chemotaktikum auf eine Fläche der Einführspitze (30) aufgetragen ist.

4. Harnkatheterprodukt nach einem der Ansprüche 2 und 3, wobei die Einführspitze (30) ein oder mehrere Reservoirs (34) beinhaltet und das Chemotaktikum innerhalb des einen oder der mehreren Reservoirs (34) enthalten ist.

5. Harnkatheterprodukt nach Anspruch 4, wobei die Einführspitze (30) zum Ausbreiten von Chemotaktikum aus dem Reservoir (34) auf den Katheter (18) konfiguriert ist, während der Katheter (18) durch die Einführspitze (30) hindurchgeht.

6. Harnkatheterprodukt nach Anspruch 5, wobei die Einführspitze (30) zum Ausbreiten von Chemotaktikum aus dem Reservoir (34) in die Harnröhre konfiguriert ist.

7. Harnkatheterprodukt nach einem der Ansprüche 1-6, ferner beinhaltend ein antimikrobielles Mittel, das dem distalen Endabschnitt (22) des Katheters (18) zugeordnet ist.

8. Harnkatheterprodukt nach einem der Ansprüche 1-7, wobei der Katheterschaft eine hydrophile Beschichtung darauf beinhaltet und das Chemotaktikum in die hydrophile Beschichtung eingebracht ist.

## Revendications

1. Produit de cathéter urinaire (16) destiné à être inséré dans l'urètre, le produit de cathéter urinaire (16) comprenant :
un cathéter (18, 40) comportant un corps de cathéter présentant une partie d'extrémité proximale (20, 42) et une partie d'extrémité distale (22, 44) ;
un agent chimiotactique disposé sur le corps du cathéter ; et
**caractérisé en ce que** :
la quantité d'agent chimiotactique disposée au niveau de l'extrémité distale (22, 44) du corps de cathéter est supérieure à une quantité d'agent chimiotactique disposée au niveau de l'extrémité proximale (20, 42) du cathéter, de sorte que le produit de cathéter est configuré pour créer un gradient chimiotactique au sein de l'urètre.

2. Produit de cathéter urinaire selon la revendication 1, dans lequel le produit de cathéter comporte en outre une pointe d'introduction (30) et l'agent chimiotactique est également associé à la pointe d'introduction.

3. Produit de cathéter urinaire selon la revendication 2, dans lequel un agent chimiotactique est appliqué sur une surface de la pointe d'introduction (30).

4. Produit de cathéter urinaire selon l'une quelconque des revendications 2 et 3, dans lequel la pointe d'introduction (30) comporte un ou plusieurs réservoirs (34) et l'agent chimiotactique est contenu au sein des un ou plusieurs réservoirs (34).

5. Produit de cathéter urinaire selon la revendication 4, dans lequel la pointe d'introduction (30) est configurée pour libérer un agent chimiotactique depuis le réservoir (34) sur le cathéter (18) lorsque le cathéter (18) passe à travers la pointe d'introduction (30).

6. Produit de cathéter urinaire selon la revendication 5, dans lequel la pointe d'introduction (30) est configurée pour libérer un agent chimiotactique depuis le réservoir (34) dans l'urètre.

7. Produit de cathéter urinaire selon l'une quelconque des revendications 1 à 6, comportant en outre un antimicrobien associé à la partie d'extrémité distale (22) du cathéter (18).

8. Produit de cathéter urinaire selon l'une quelconque des revendications 1 à 7, dans lequel le corps de cathéter comporte un revêtement hydrophile, et l'agent chimiotactique est chargé dans le revêtement hydrophile.
